# EUROPEAN PATENT SPECIFICATION

(11) **EP 0 625 759 B1**
(45) Date of publication and mention of the grant of the patent: **29.08.2001**
(21) Application number: 94105918.0
(22) Date of filing: 16.04.1994
(51) Int. Cl.: G06F 19/00, G11B 7/00

(54) **Medical information management system**
System zur Verwaltung medizinischer Daten
Système de gestion des informations médicales

(30) Priority: 20.04.1993 JP 11528393
(43) Date of publication of application: 23.11.1994
(73) Proprietor: NIPPON CONLUX CO., LTD., Chiyoda-ku Tokyo-To (JP); Shiina, Shinichi, Tokyo-to (JP)
(72) Inventor: Honda, Hiroto, Tsurugashima-shi, Saitama-ken (JP)
(74) Representative: Selting, Günther, Dipl.-Ing.

(56) References cited:
- EP-A- 0 467 693
- FR-A- 2 664 730
- GB-A- 2 228 821
- US-A- 4 991 581
- PROCEEDINGS OF THE FIFTH ANNUAL IEEE SYMPOSIUM ON COMPUTER-BASED MEDICAL SYSTEMS, June 1992, DURHAM, NORTH CAROLINA, USA pages 156 - 169 FRANCHI ET AL 'Multimedia Perspectives for Next Generation PAC Systems'
- DATABASE INSPEC INSTITUTE OF ELECTRICAL ENGINEERS, STEVENAGE, GB Inspec No. AN3980074 June 1991, MALONEY ET AL 'Multimedia object file design for medical images' & MUG QUARTERLY, vol.21, no.3, June 1991, USA pages 57 - 60

## Description

The present invention generally relates to a medical information management system which records a patient's personal medical information onto an optical card for various management purposes.

Medical information management systems are popularly known, in which various character and image information such as characters, drawing, X-ray photograph and computer tomogram (so-called CT) originally entered in patients' case records (charts) as their personal medical information are optically or magnetically recorded onto recording media such as optical cards and magnetic disks.

Because the medical image information, in general, must be highly detailed and precise, recording, for example, a single X-ray photograph as a piece of image information usually requires a storage capacity of about one megabyte. In addition, in order for a doctor to obtain knowledge about or diagnose the morbid state of a given patient, image information corresponding to at least three X-ray photographs would be necessary. This means that a medium for recording the patient's medical information must have storage capacity of more than three megabytes.

Nevertheless, the storage capacity of today's commercially available optical cards is only about three megabytes at the most, and thus, even if some form of data compression technique etc. are employed, it is very difficult or impossible for the optical cards to record thereon more than image information about two or three X-ray photographs and character information (and/or drawing information) about the patient's personal identification, clinical history, doctor's observation and the like.

Further, in the case of a patient having a circulatory disease, the doctor may more easily obtain knowledge of or diagnose the morbid state of the patient by listening to the patient's characteristic cardiac sounds, cardiac murmurs etc., rather than by examining the patient's X-ray photograph. However, since the prior medical information management systems only express such patient's cardiac sounds, cardiac murmurs etc. in onomatopoeic words or in schematic diagram and then record these as character and image information, it is very difficult to promptly obtain knowledge about the patient's morbid state just by examining the thus-recorded character or image information.

EP 0 467 693 A3 discloses a medical information management system for recording a patient's personal medical information onto an optical memory card to manage the medical information. The information management system comprises a read/write means for writing information obtained from a patient in letters or images on the optical memory card in accordance with a predetermined format and outputs the recorded medical information from the optical memory card whenever necessary for producing the information on a monitor screen and in the form of film. This information system does not record any sound.

From DATABASE INSPEC INSTITUTE OF ELECTRICAL ENGINEERS, Stevenage, GB, Inspec No. AN3980074 June 1991, MALONEY ET AL "Multimedia object file design for medical images", abstract & MUG QUARTERLY, vol. 21, no. 3, June 1991, USA, pages 57-60, there is known a computer system for storing images that is also capable of storing echocardiograph sequences, EKGs, radiographic images and heart murmurs.

PROCEEDINGS OF THE FIFTH ANNUAL IEEE SYMPOSIUM ON COMPUTER-BASED MEDICAL SYSTEMS, June 1992, DURHAM, NORTH CAROLINA, USA, pages 156-169, FRANCHI ET AL "Multimedia Perspectives for Next Generation PAC Systems", page 160, lines 1-11, page 165, last paragraph, discloses an architecture of a multimedia medical information system that is capable of storing voice in addition to other patient's data.

FR-2-664 730 discloses a CD-ROM-like disk for storing images and sound in an interlaced manner.

US-A-4 991 581 describes an apparatus of recording and processing body sounds for assessment of the sound and possible diagnosis of any abnormalities associated with this sound. The sound is displayed on a display means as a visual sound waveform diagram.

It is an object of the present invention to provide an improved medical information management system which is capable of efficiently recording medical information corresponding to a patient's morbid state onto an optical memory medium having limited storage capacity.

The medical information management system of the present invention is defined by claim 1, and the optical memory medium used in it in claim 10.

In a most preferred embodiment of the present invention, the optical memory medium is an optical card. The optical card is a nonvolatile memory which is very handy for carrying, has sufficiently large storage capacity (about three megabytes) and is very easy to write data thereon. For these reasons, the optical card is known as one of the most suitable recording media for recording patient's personal medical information. Thus, by using such an optical card as a recording medium, it is possible to efficiently record medical information as needed depending on a specific morbid state of the patient. Therefore, if only the patient carries the optical card bearing his or her medical information to a given doctor, the doctor, by only setting the optical card in the read section, can read the patient's medical information, can have the character and image information visually shown and also can have the sound information audibly reproduced or sounded. This allows the doctor to readily obtain knowledge about the patient's morbid state.

The doctor can easily acquire or diagnose the patient's morbid state by listening to the patient's characteristic cardiac sounds and murmurs, particularly to the cardiac murmurs if the patient has a circulatory disease. Similarly to the cardiac murmurs, the respiratory or breathing sounds are very characteristic of, i.e., very faithfully reflects a disease. Thus, it is desirable to record the respiratory sounds as sound information so that the doctor can easily obtain knowledge about the patient's morbid state by audibly reproducing the information with the present system.

In the most preferred embodiment of the present invention, the sound information may be graphed as a visual sound waveform diagram as well as being audibly reproduced through the reproduction section. This allows the doctor to more clearly acquire the patient's morbid state both aurally and visually.

In addition, even when the patient goes to a hospital for the first time, the patient can let a doctor of the hospital readily know about his or her morbid state by only presenting the medical information bearing optical card and having it set in the read section of the system. Further, since only desired medical information depending on his or her own morbid state may be recorded on the private optical card and since it sometimes may not be necessary to record image information of a large data amount on the card, it is very often sufficient for the patient to carry only one optical card. This is far more economical than when the patient carries several optical cards.

Moreover, even in medical educational organizations, the medical information management system of the present invention can be applied as an effective means for morbid state acquisition, by reproducing the sound information recorded on the optical card.

The preferred embodiment of the present invention will be described in detail below with reference to the accompanying drawings.

In the accompanying drawings:
Fig. 1 is a block diagram illustrating the general structure of a medical information management system in accordance with an embodiment of the present invention;
Fig. 2 is a top plan view illustrating an example of an optical card for use with the medical information management system of Fig. 1;
Fig. 3 is a diagram illustrating an example of recorded data on the optical card;
Fig. 4 is a diagram explanatory of a display state on a display device of Fig. 1;
Fig. 5 is a flowchart illustrating an example of a write process carried out by the medical information management system of Fig. 1; and
Fig. 6 is a flowchart illustrating an example of a read process carried out by the medical information management system of Fig. 1.

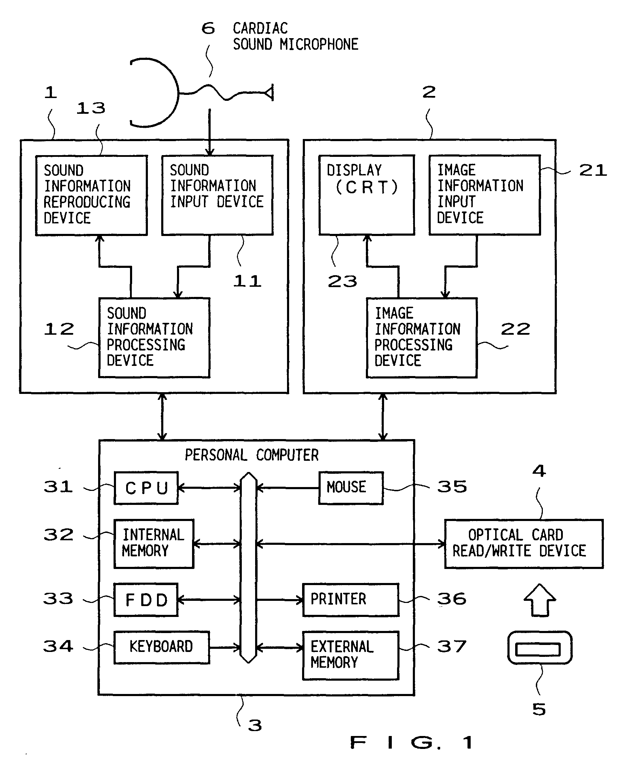

Fig. 1 is a block diagram illustrating the general structure of a medical information management system in accordance with a preferred embodiment of the present invention. This management system generally comprises sound an information processing device 1, an image information processing device 2, a personal computer 3, an optical card read/write device 4 and an optical card 5 that is removably attached to or set in the optical card read/write device 4.

The sound information processing device 1 is composed of a sound information input device 11, a sound information processing board 12 and a sound information reproducing device 13.

The sound information input device 11, which includes a cardiac sound microphone 6, converts patient's cardiac sounds, cardiac murmurs or respiratory sounds into respective analog sound information signals, and then outputs the sound information to the sound processing board 12.

The sound information reproducing device 13, which may for example comprise a speaker, audibly reproduces the analog sound information signals received from the sound information processing board 12.

The image information processing device 2 is composed of an image information input device 21, an image information processing board 22 and a display device 23.

The image information input device 21, which includes a video camera or an FD camera (an electronic camera with a floppy disk drive), generates NTSC signals based on X-ray photograph information and outputs the NTSC signals to the image information processing board 22.

The image information processing board 22 converts the NTSC signals received from the image information input device 21 into digital image information and outputs the digital image information to the personal computer 3. The image information processing board 22 also converts digital image information received from the personal computer 3 into NTSC signals and outputs the NTSC signals to the display device 23. In addition, the image information processing board 22 receives digital sound information that is being provided from the personal computer 3 to the sound information processing board 12 of the sound information input device 11 and converts the input sound information into a sound waveform diagram in the form of NTSC signals. The sound waveform diagram is provided to the display device 23.

The display device 23 comprises a TV monitor such as a CRT display and visibly presents thereon the NTSC signals provided from the image information processing board 22. Namely, the image information processing board 22 outputs NTSC signals based on normal image information of X-ray photograph or the like and based on a sound waveform diagram that visually represents digital sound information, and the display device 23 presents thereon visual images corresponding to such information. For the sound waveform diagram, the display device 23 presents a corresponding graph.

The personal computer 3 controls the entire operation of the medical information management system, and it basically comprises a CPU 31, internal memories (ROM and RAM) 32, peripherals and I/O interfaces. As such peripherals, this personal computer 3 includes a floppy disk drive (FDD) 33, a keyboard 34, a mouse 35, a printer 36, an external memory (e.g. hard disk), etc. Further, the sound information processing board 12 and image information processing board 22 are attached, for example, to extension slots in the personal computer 3. Accordingly, the personal computer 3 also incorporates therein software programs that are necessary for executing various processes on the sound and image information by means of the sound information processing board 12 and image information processing board 22.

The optical card read/write device 4, which is connected to the personal computer 3 as one of the peripherals thereof, writes and reads data to and from the optical card 5 under the control of the personal computer 3.

The medical information management system which, as mentioned above, comprises the sound information processing device 1, image information processing device 2, personal computer 3 and optical card read/write device 4 is installed within a hospital. The optical card 5 is possessed by an individual patient and is set in the optical card read/write device 4 such as when the patient receives medical examination by a doctor.

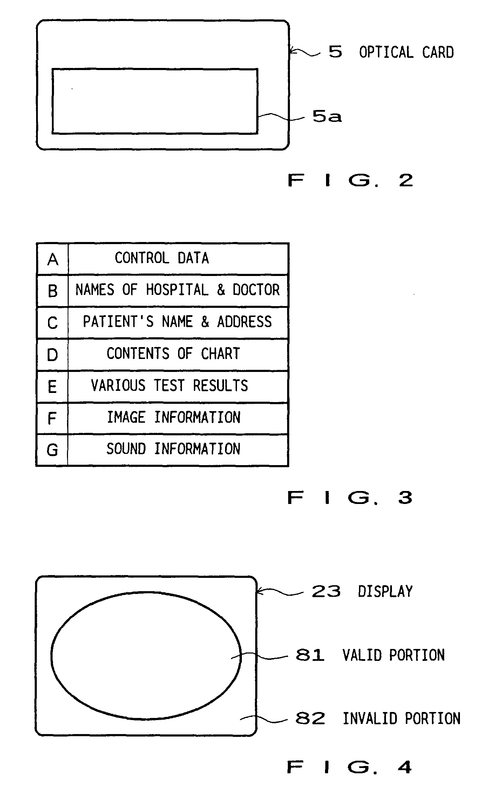

As typically shown in Fig. 2, the optical card 5 is a rectangular-shaped card having a size of about 85.6 mm length and about 54 mm width and has a thickness of about 0.76 mm. A predetermined area 5a on the card surface is a recording area, onto which digital data corresponding to the patient's medical information are optically recorded. The recorded digital data are then optically read out from the card when needed. Such an optical card 5 may for example be a DELA-standard card available from Drexler Inc. The optical card read/write device 4, which is designed to allow removable setting therein of the optical card 5, can optically write desired information (in this example, medical information comprising character, image, sound information etc.) onto the thus-set card 5 using light beams and read out the written information as needed.

In the case of the DELA-standard optical card, every new information is written as an addition onto unrecorded portion of the recording area (in a write-once fashion). Specific examples of the optical card read/write device 4 and optical card 5 are disclosed, for example, in Japanese Patent Laid-open Publication Nos. SHO 58-500437, 59-195327, 61-48135, 61-137245, 62-256248, 62-262239 and 62-266748, and therefore a detailed description on these is omitted herein.

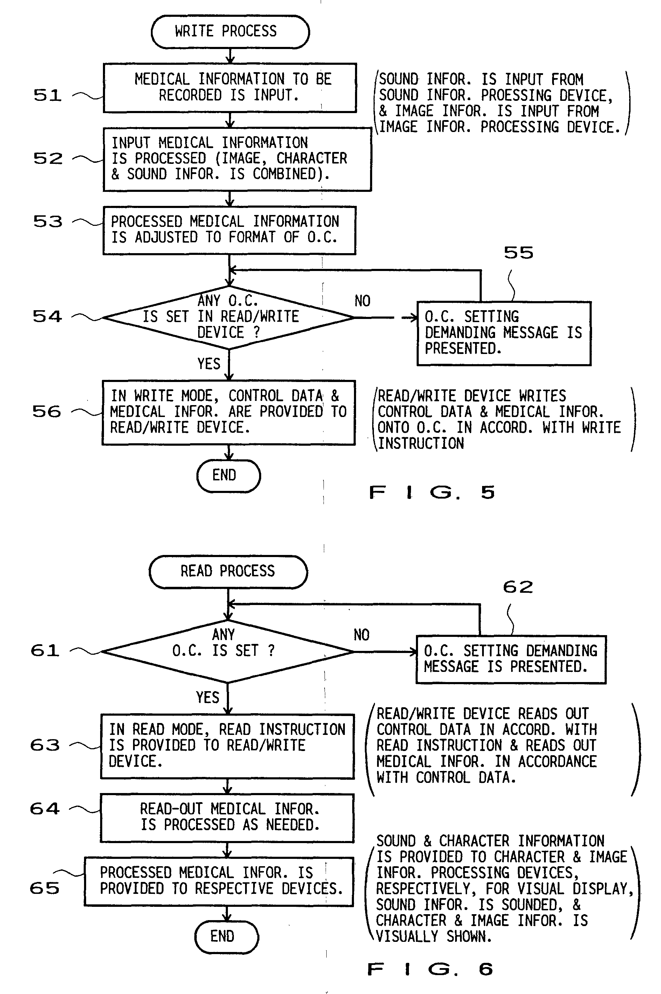

Fig. 3 illustrates an example of the medical information stored on the optical 1 card 5. As illustrated, the recording area 5a of each optical card 5 is divided into a plurality of memory regions A - G for recording a plurality of data. On memory region A are stored control data that are indicative of the addresses and storage capacities of the individual memory regions. On memory region B, information such as the names of the hospital and doctor in charge of the patient is stored as character information. Further, on memory region C, personal information such as the patient's name and age is stored as character information. Further, on memory region D, contents of the patient's chart such as results of the doctor's questioning and diagnosis are stored as character information. Further, on memory region E, results of various tests such as urine analysis, blood type and blood pressure are stored as character information. Further, on memory region F is stored image information such as X-ray photograph and CT. Finally, on memory region G is stored sound information such as cardiac murmurs and respiratory sound detected by the above-mentioned cardiac sound microphone 6.

The hospital name, patient's disease name and blood type and results of various tests may be directly stored as character data. But, in order to save storage capacity, it is desirable that these data are stored in encoded form, in which case the encoded data are decoded by the personal computer 3 for subsequent necessary processing. The sound information may be PCM data obtained by subjecting analog sound waveform signals to a digital PCM conversion; however, this approach will require large storage capacity, and accordingly only a limited amount of the sound information can be recorded on a single optical card 5. It should be appreciated that, in order to save the storage capacity, the sound information may of course be stored in some data-compressed form by the use of DPCM, ADPCM or any other suitable sound data compression technique.

Because of this, the embodiment attempts to minimize the required storage capacity by inserting the sound and/or character information in an invalid portion of the image information. For instance, as shown in Fig. 4, if image information generally of an elliptical shape is presented on the display device 23, the doctor only needs the valid portion 81 in order to acquire the patient's morbid state and does not need an invalid portion 82 surrounding the valid portion 81. Therefore, the sound and/or character information can be inserted in the invalid portion 82 without disturbing the valid portion 81 and thus, the storage capacity for the image information can be utilized efficiently. More specifically, in such a case where one frame of image information data is stored in a predetermined storage area of the optical card 5, it is possible that, instead of the image information being stored in the entire frame storage area, image information representing the valid portion 81 is stored in a part of the area corresponding to the valid portion 81, and sound information and/or character information is stored in the other part of the area corresponding to the invalid portion 82.

Now, an example of a write process carried out by the personal computer 3 for writing medical information onto the optical card 5 will be described with reference to a flowchart of Fig. 5.

Step 51: Medical information (character, image and sound information) of a given patient to be recorded onto the optical card 5 is fetched: the character information is input into directly from the keyboard 34; the image information is input from the image information processing device 2 as digital image information; and the sound information is input from the sound information processing device 1 as digital sound information.

Step 52: Any data processes necessary are applied to the respective medical information input in the manner mentioned above. For example, sound components other than the patient's cardiac sounds, cardiac murmurs and respiratory sounds may be filtered out, the sound and character information may be inserted in the invalid portion 82 of the image information as shown in Fig. 4, and/or, the image, character and sound information may be stored in a combined or composite form.

Step 53: The medical information as processed in step 52 above is adjusted to the recording format of the optical card.

Step 54: It is determined whether or not any optical card 5 is attached to or set in the optical card read/write device 4. The personal computer 3 goes to step 55 if the optical card 5 is not set, but goes to step 56 if the optical card 5 is set.

Step 55: Because off the negative determination in step 54 above, a message that demands setting of the optical card 5 is presented on the display device 23 or given in voice or the like.

Step 56: After preparation of the medical information to be written and control data indicating how to write the medical information is completed, the personal computer 3 waits for a write instruction signal to be input by operation of the keyboard 34 etc. Then, when the write instruction signal is input, the personal computer accesses the optical card read/write device 4 so as to write, onto the optical card 5, the medical information and control data that have been input previously. That is, when the write instruction signal is input, the personal computer 3 changes the operation mode into the optical card write mode and provides the control data and medical information to the optical card read/write device 4. The optical card read/write device 4 in turn writes the medical information and control data onto the optical card 5 in accordance with the write instruction signal. Namely, this write process writes the medical information and control data as addition to an unrecorded portion of the card rather than as a rewrite to an already recorded portion of the card. However, the medical information and control data may of course be written as a rewrite to the already written portion if a rewritable recording medium is employed.

An example of a read process performed for the personal computer 3 to read out the medical information from the optical card will be described with reference to the flowchart shown in Fig. 6.

Step 61: A determination is made as to whether any optical card 5 is set in the optical card read/write device 4. The process goes to step 62 if the answer is in the negative, but it goes to step 63 if the answer is in the affirmative indicating that an optical card 5 is set in the read/write device 4.

Step 62: Because of the determination in the preceding step 61 that no optical card is set in the optical card read/write device 4, a message for demanding setting of an optical card is given on the display device 23 or in other suitable form such as vocal sound.

Step 63: When medical information to be read out is specified by the user's operation of the keyboard 34 or mouse 35, the personal computer 3 changes the operation mode into the optical card read mode and then provides the optical card read/write device 4 with a read instruction signal corresponding to the medical information to be read out. In response to the read instruction signal, the read/write device 4 reads out the control data from the optical card 5 and then reads out medical information in accordance with the read instruction signal.

Step 64: Any data processes necessary are applied to the medical information read out from the optical card 5. For example, the character, sound and image information may be extracted out from the medical information that has been so far stored in synthesized form as shown in Fig. 4.

Step 65: If the medical information processed in the preceding step 64 is the sound information, the sound information is provided to the sound information processing device 1; if the medical information processed in the preceding step 64 is the character or image information, the character or image information is provided to the image information processing device 2. Thus, the sound information processing device 1 sounds or audibly reproduces the provided sound information. On the other hand, the image information processing device 2 visually shows the provided character or image information on the display device 23. If both of the character and image information are provided to the image information processing device 2, the character information may be shown with the image information in a superimposed manner. Further, if the medical information processed in the preceding step 64 is the sound information and if it is desired to show a corresponding sound waveform diagram on the display 23 with the sound information audibly reproduced, image information on the waveform diagram is provided to the image information processing device 13. In this case, the sound waveform diagram can be stored as image information in the optical card 5; however, in order to save the storage capacity of the card 5, it is more preferable that sound information is temporarily stored in data buffer memory to thereby prepare a sound waveform diagram and then the sound waveform diagram is displayed while sound corresponding to the sound information stored in the buffer memory is audibly reproduced.

According to the embodiment so far described, the optical cards of the write-once-type are employed such that any data, once written, will not disappear, i.e., any previously written information will not be erased by overwriting, as opposed to magnetic recording media typically represented by cassette tapes. In addition, the optical cards are not affected by magnetism, static electricity, radiation etc. and therefore have better data storage capability than other types of recording media. Further, even when the optical cards have been soiled, they can readily be used again by wiping their surfaces.

In addition, as opposed to tape-shape recording media with which a considerable long time is taken to retrieve desired data therefrom, the optical cards allow direct and quick access to desired data in conjunction with the personal computer. For example, names of recorded data may be shown on the display screen of the personal computer so that, in response to the user's selection of a desired data name shown on the screen, the corresponding recorded sound information can be quickly read out for the audible and/or visual reproduction.

The optical cards are generally of a credit card size and therefore can be attached to the patients' charts or the like so as to be easily filed in a desired place. Furthermore, the optical cards are very handy for carrying, and thus if the patient goes to a doctor of another hospital or the like, the patient can have his or her clinical history readily acquired by only presenting the optical card.

Moreover, because data are stored on the optical cards in coded form and thus are difficult to be altered or decoded by any unauthorized person, the optical cards can provide reliable protection of patients' privacy and also can provide superior data safety.

Moreover, because the optical cards can record not only sound information but also image and character information, they provide easy communication and storage of various kinds of information independently.

Although the above embodiment has been described in connection with such a case where cardiac sounds and murmurs detected by a cardiac sound microphone are recorded and reproduced, pulse wave detected by a pulse wave detector, electrocardiogram waveform detected by an electrocardiograph, cardiac echo diagram detected by an ultrasonic cardiac diagnosis device, cardiac tomogram etc. may be recorded as image signals for being graphed as waveform diagram. Moreover, the cardiac sound microphone may be replaced by an electrostethophone utilizing the Doppler effect.

As apparent from the foregoing, the present invention permits recording of medical information corresponding to a patient's morbid state to be efficiently recorded onto an optical card that has a limited storage capacity.

## Claims

1. A medical information management system for recording a patient's personal medical information onto an optical memory medium (5) to manage the medical information, said optical memory medium (5) being portable and used as the patient's private memory medium, said medical information system comprising:
write means (3,4,6,11) for writing onto said memory medium (5) any information selected from among character, image and sound information corresponding to a morbid state of the patient as the medical information,
read means (3,4) for optically reading out the medical information written on said optical memory medium (5),
display means (23) for visually presenting the character and image information of the medical information read out by said read means, and
reproduction means (13) for audibly reproducing the sound information of the medical information read out by said read means (3,4),
wherein said write means (3,4) inserts at least part of data of said sound information between data of the image information to form composite data, so as to write the information as the composite data onto said memory medium (5), and
said read means (3,4) retrieves said composite data from said memory medium (5) and reads out the sound and image information separately by separating the at least part of data of at least the sound information and the data of the image information from said composite data, and
wherein said write means (3,4) writes the at least part of the sound information onto a predetermined part of a storage area of said memory medium (5) provided for storing one frame of the image information data, said predetermined part of the storage area substantially corresponding to a peripheral portion of the frame and being a part where no substantially valid image information is stored.

2. A medical information management system as defined in claim 1 wherein said write means (3,4) further inserts at least part of data of the character information between the data of the image information to form said composite data, so as to write the information as the composite data onto said memory medium (5).

3. A medical information management system as defined in claim 2 wherein said write means (3,4) writes the at least part of the character information onto a predetermined part of a storage area of said memory medium (5) provided for storing one frame of the image information data, said predetermined part of the storage area being a part where no substantially valid image information is stored.

4. A medical information management system as defined in one of claims 1-3 wherein said display means includes printer means.

5. A medical information management system as defined in one of claims 1-4 wherein said display means presents a visual sound waveform diagram corresponding to the sound information reproduced by said reproduction means.

6. A medical information management system as defined in claim 4 which further comprises means for preparing the visual sound waveform diagram on the basis of the sound information read out by said read means (3,4).

7. A medical information management system as defined in one of claims 1-6 wherein said optical memory medium is an optical card (5).

8. A medical information management system as defined in one of claims 1-7 wherein said sound information represents any of the patient's cardiac sound and cardiac murmur.

9. A medical information management system as defined in one of claims 1-8 wherein said sound information represents the patient's breathing sound.

10. An optical memory medium for use in a medical information management system of one of claims 1-9, said optical memory medium being a portable card-like medium (5) independently provided for the patient and capable of being removably attached to a write device, said memory medium comprising data areas storing information on the patient's name and address, storing information on the patient's clinical record, storing information on results of various tests performed on the patient, storing image information representing a diagnosis on the patient and storing sound information representing a diagnosis on the patient, respectively, at least a part of said sound information being written onto a predetermined part of a storage area of said memory medium (5) provided for storing one frame of the image information data, said predetermined part of the storage area substantially corresponding to a peripheral portion of the frame and being a part where no substantially valid image information is stored.

11. An optical memory medium as defined in claim 10 wherein said sound information represents any of the patient's cardiac sound, cardiac murmur and breathing sound.

## Patentansprüche

1. Medizinisches Informationsmanagementsystem zum Aufzeichnen der persönlichen medizinischen Informationen eines Patienten auf einem optischen Speichermedium (5) zur Verwaltung der medizinischen Informationen, wobei das optische Speichermedium (5) tragbar ist und als persönliches Speichermedium des Patienten verwendet wird und das medizinische Informationsmanagementsystem aufweist:
eine Schreibeinrichtung (3,4,6,11) zum Aufzeichnen von aus Zeichen, Bild und Schall bestehenden Informationen über den Krankheitszustand des Patienten als medizinische Information auf das Speichermedium (5),
eine Leseeinrichtung (3,4) zum optischen Auslesen der auf das optische Speichermedium (5) geschriebenen medizinischen Informationen,
eine Anzeigeeinrichtung (23) zur visuellen Darstellung der Zeichen- und Bildinformationen der von der Leseeinrichtung ausgelesenen medizinischen Informationen, und
eine Wiedergabeeinrichtung (13) zur akustischen Wiedergabe der Schallinformation der von der Leseeinrichtung (3,4) ausgelesenen medizinischen Informationen,
wobei die Schreibeinrichtung (3,4) zur Bildung einer Datenzusammenstellung mindestens einen Teil der Daten der Schallinformation zwischen die Daten der Bildinformation schiebt, so dass die Informationen als Datenzusammenstellung auf das Speichermedium (5) geschrieben werden, und
die Leseeinrichtung (3,4) die Datenzusammenstellung vom Speichermedium (5) abruft und die Schall- und Bildinformationen derart getrennt ausliest, dass mindestens ein Teil der Daten mindestens der Schallinformation und die Daten der Bildinformation von der Datenzusammenstellung getrennt werden, und
wobei die Schreibeinrichtung (3,4) den mindestens einen Teil der Schallinformation auf einen zum Speichern eines Einzelbilds der Bildinformationsdaten vorgesehenen vorbestimmten Teil eines Speicherbereichs des Speichermediums (5) schreibt, wobei der vorbestimmte Teil des Speicherbereichs im wesentlichen einem Randgebiet des Einzelbilds entspricht und ein Teil ist, in dem keine im wesentlichen gültigen Bildinformationen gespeichert sind.

2. Medizinisches Informationsmanagementsystem nach Anspruch 1, bei dem die Schreibeinrichtung (3,4) zwecks Datenzusammenstellung ferner mindestens einen Teil der Zeicheninformation zwischen die Daten der Bildinformation schiebt, so dass die Informationen als Datenzusammenstellung auf das Speichermedium (5) geschrieben werden.

3. Medizinisches Informationsmanagementsystem nach Anspruch 2, bei dem die Schreibeinrichtung (3,4) mindestens den Teil der Zeicheninformation auf einen zum Speichern eines Einzelbilds der Bildinformationsdaten vorgesehenen vorbestimmten Teil des Speicherbereichs des Speichermediums (5) schreibt, wobei der vorbestimmte Teil des Speicherbereichs ein Teil ist, in dem keine im wesentlichen gültigen Informationen gespeichert sind.

4. Medizinisches Informationsmanagementsystem nach einem der Ansprüche 1-3, bei dem die Anzeigeeinrichtung eine Druckeinrichtung aufweist.

5. Medizinisches Informationsmanagementsystem nach einem der Ansprüche 1-4, bei dem auf der Anzeigeeinrichtung ein sichtbares Schallwellendiagramm entsprechend den von der Wiedergabeeinrichtung wiedergegebenen Schallinformation abgebildet wird.

6. Medizinisches Informationsmanagementsystem nach Anspruch 4, das ferner eine Einrichtung zur Erstellung des sichtbaren Schallwellendiagramms auf der Basis der von der Leseeinrichtung (3,4) ausgelesenen Schallinformation aufweist.

7. Medizinisches Informationsmanagementsystem nach einem der Ansprüche 1-6, bei dem das optische Speichermedium eine optische Karte (5) ist.

8. Medizinisches Informationsmanagementsystem nach einem der Ansprüche 1-7, bei dem die Schallinformationen die Herztöne oder das Herzgeräusch des Patienten darstellen.

9. Medizinisches Informationsmanagementsystem nach einem der Ansprüche 1-8, bei dem die Schallinformationen das Atemgeräusch des Patienten darstellen.

10. Optisches Speichermedium für ein medizinisches Informationsmanagementsystem nach einem der Ansprüche 1-9, wobei das optische Speichermedium ein tragbares kartenartiges Medium (5) ist, das für den betreffenden Patienten persönlich vorgesehen ist und abnehmbar an einer Schreibvorrichtung befestigt werden kann, wobei das Speichermedium aufweist: Datenbereiche, in denen Informationen bezüglich des Namens und der Adresse des Patienten, des klinischen Befunds des Patienten, Ergebnissen verschiedener am Patienten vorgenommener Tests, eine Patientendiagnose darstellender Bildinformationen bzw. eine Patientendiagnose darstellender Schallinformationen gespeichert werden, wobei mindestens ein Teil der Schallinformation auf einen zum Speichern eines Einzelbilds der Bildinformationsdaten vorgesehenen vorbestimmten Teil eines Speicherbereichs des Speichermediums (5) geschrieben wird, wobei der vorbestimmte Teil des Speicherbereichs im wesentlichen einem Randgebiet des Einzelbilds entspricht und ein Teil ist, in dem keine im wesentlichen gültigen Informationen gespeichert sind.

11. Optisches Speichermedium nach Anspruch 10, bei dem die Schallinformationen die Herztöne, das Herzgeräusch oder das Atemgeräusch des Patienten darstellen.

## Revendications

1. Système de gestion d'informations médicales pour enregistrer les informations médicales personnelles d'un patient sur un support à mémoire optique (5) pour gérer ces informations médicales, ledit support à mémoire optique (5) étant portable et utilisé à titre de support à mémoire privé du patient, ledit système d'informations médicales comprenant :
des moyens d'écriture (3,4,6,11) pour écrire sur ledit support à mémoire (5), à titre d'informations médicales, des informations quelconques choisies parmi des informations au format caractère, image et sonore correspondant à un état morbide du patient,
des moyens de lecture (3,4) pour lire optiquement les informations médicales écrites sur ledit support à mémoire optique (5),
des moyens d'affichage (23) pour présenter visuellement les informations au format caractère et image des informations médicales lues par lesdits moyens de lecture, et
des moyens de reproduction (13) pour reproduire de manière audible les informations au format sonore des informations médicales lues par lesdits moyens de lecture (3,4),
dans lequel lesdits moyens d'écriture (3,4) insèrent au moins une partie des données desdites informations au format sonore entre des données des informations au format image pour former des données composites, de manière à écrire les informations sous la forme des données composites sur ledit support à mémoire (5), et
lesdits moyens de lecture (3,4) extraient lesdites données composites dudit support à mémoire (5) et lisent les informations au format sonore et image séparément, en séparant desdites données composites ladite au moins une partie des données d'au moins les informations au format sonore et les données des informations au format image, et
dans lequel lesdits moyens d'écriture (3,4) écrivent ladite au moins une partie des informations au format sonore sur une partie prédéterminée d'une zone d'enregistrement dudit support à mémoire (5) prévue pour enregistrer une trame des données d'informations au format image, ladite partie prédéterminée de la zone d'enregistrement correspondant sensiblement à une partie périphérique de la trame et constituant une partie sur laquelle aucune information au format image sensiblement valide n'est enregistrée.

2. Système de gestion d'informations médicales selon la revendication 1, dans lequel lesdits moyens d'écriture (3,4) insèrent, en outre, au moins une partie des données des informations au format caractère entre les données d'informations au format image pour former lesdites données composites, de manière à écrire les informations sous la forme des données composites sur ledit support à mémoire (5).

3. Système de gestion d'informations médicales selon la revendication 2, dans lequel lesdits moyens d'écriture (3,4) écrivent ladite au moins une partie des informations au format caractère sur une partie prédéterminée d'une zone d'enregistrement dudit support à mémoire (5) prévue pour enregistrer une trame des données d'informations au format image, ladite partie prédéterminée de la zone d'enregistrement constituant une partie sur laquelle aucune information au format image sensiblement valide n'est enregistrée.

4. Système de gestion d'informations médicales selon les revendications 1 à 3, dans lequel lesdits moyens d'affichage comprennent des moyens d'imprimante.

5. Système de gestion d'informations médicales selon l'une quelconque des revendications 1 à 4, dans lequel lesdits moyens d'affichage présentent un diagramme d'ondes sonores visuel correspondant aux informations au format sonore reproduites par lesdits moyens de reproduction.

6. Système de gestion d'informations médicales selon la revendication 4 qui comprend, en outre, des moyens pour préparer le diagramme d'ondes sonores visuel sur la base des informations au format sonore lues par lesdits moyens de lecture (3,4).

7. Système de gestion d'informations médicales selon l'une quelconque des revendications 1 à 6, dans lequel ledit support à mémoire optique est une carte optique (5).

8. Système de gestion d'informations médicales selon l'une quelconque des revendications 1 à 7, dans lequel lesdites informations au format sonore représentent l'un quelconque des bruits du coeur et des souffles cardiaques du patient.

9. Système de gestion d'informations médicales selon l'une quelconque des revendications 1 à 8, dans lequel lesdites informations au format sonore représentent le bruit respiratoire du patient.

10. Support à mémoire optique utilisable dans un système de gestion d'informations médicales selon l'une quelconque des revendications 1 à 9, ledit support à mémoire optique étant un support portable en forme de carte (5) fourni indépendamment au patient et qui peut être raccordé de manière amovible à un dispositif d'écriture, ledit support à mémoire comprenant des zones de données enregistrant des informations concernant le nom et l'adresse du patient, enregistrant des informations concernant le dossier médical du patient, enregistrant des informations concernant les résultats des divers examens effectués sur le patient, enregistrant des informations au format image représentant un diagnostic concernant le patient et enregistrant des informations au format sonore représentant un diagnostic concernant le patient, respectivement, une partie au moins desdites informations au format sonore étant écrite sur une partie prédéterminée d'une zone d'enregistrement dudit support à mémoire (5) prévue enregistrant une trame des données d'informations au format image, ladite partie prédéterminée de la zone d'enregistrement correspondant sensiblement à une partie périphérique de la trame et constituant une partie sur laquelle aucune information au format image sensiblement valide n'est enregistrée.

11. Support à mémoire optique selon la revendication 10, dans lequel lesdites informations au format sonore représentent l'un quelconque des bruits du coeur, souffles cardiaques et bruits respiratoires du patient.
